# EUROPEAN PATENT APPLICATION

(11) **EP 1 201 197 A1**
(43) Date of publication of application: **02.05.2002**
(21) Application number: 00121016.0
(22) Date of filing: 27.09.2000
(51) Int. Cl.: A61B 17/70

(54) **Spinal fixation hook and spinal fixation device**

(71) Applicant: Yeh, Chung-Chun, Taipei (TW)
(72) Inventor: Yeh, Chung-Chun, Taipei (TW)
(74) Representative: Grünecker, Kinkeldey, Stockmair & Schwanhäusser Anwaltssozietät

(57) **Abstract**

A spinal fixation device includes a fixation seat (200), a rod-shaped object (150,400), one or more fixation hooks (100), and one or more fixation elements (310,315,320). The fixation seat is provided with at least two through holes (210,220). The rod-shaped object is put through one of the through holes of the fixation seat. The fixation hooks comprise an extension rod (150) and a hooked portion (110,110'). The extension rod is put through a through hole of the fixation seat. The fixation elements are intended to fix the rod-shaped object and the fixation hook in the through holes of the fixation seat.

## Description

### FIELD OF THE INVENTION

The present invention relates to a spinal fixation hook and a spinal fixation retrieval device.

### BACKGROUNF OF THE INVENTION

The conventional spinal fixation hook has a hooked portion with a threaded portion integrally therewith, such as those made by J.B.S Corporation and Depuy corporation of the United States. Another conventional spinal fixation hook has a fixation seat to facilitate the fastening of the hook with a fixation rod by a screw. These hooks are made respectively by Acro.Med Corp. of the United States, the Sofamor Company of France, the Peter Brehm Co. of Germany, the G. Cremascoli Co. of Italy, and the Biomet Co. from Europe. The fixation hook made by Danek Group Inc., Medical Division, of the United States is provided with a modular fixation seat or the TSRH fixation seat for fastening with a fixation rod. In the use of such conventional fixation hooks as described above, the fixation rod is placed at a level higher than that of the hooked portion of the spinal fixation hook. In the meantime, the fixation mechanism for fixing the fixation hook to the fixation rod is located at the level higher than that of the fixation rod. If such conventional fixation hooks are used as partial elements or members of a spinal fixation retrieval device, the spinal fixation retrieval device will protruded from the vertebra to be fixed with a undesired height. Such a shortcoming as described above is well acknowledged by the medical practitioners. There is no remedial measure yet. The present invention is intended to provide a solution to the problems described above.

### SUMMARY OF THE INVENTION

The primary objective of the present invention is to provide a spinal fixation hook devoid of a fixation means per se.

It is another objective of the present invention to provide a spinal fixation retrieval device which has a fixation hook devoid of a fixation means per se.

A spinal fixation hook constructed according to the present invention comprises an extension rod and a hooked portion at one end of said extension rod. Said extension rod is preferably a straight rod.

Preferably, said hooked portion has a U shape, an S shape, a shape like the U shape, or a shape like the S shape.

In light of the spinal fixation hook of the present invention being free from the fixation means, the fixation rod to which the spinal fixation hook is fixed is about equal to the height of the extension rod of the spinal fixation hook, or the height of the vertebra. As a result, the present invention has overcome the shortcoming of the conventional spinal fixation hooks. In addition, the spinal fixation hook of the present invention simplifies the construction of the spinal fixation retrieval device. The conventional spinal fixation retrieval device is relatively complicated in construction.

### BRIEF DESCRIPTION OF THE DRAWINGS

FIG. 1 shows a schematic perspective view of a spinal fixation hook of a preferred embodiment of the present invention.
FIGS. 2-8 are schematic perspective views of spinal fixation hooks of other preferred embodiments of the present invention.

### DETAILED DESCRIPTION OF THE INVENTION

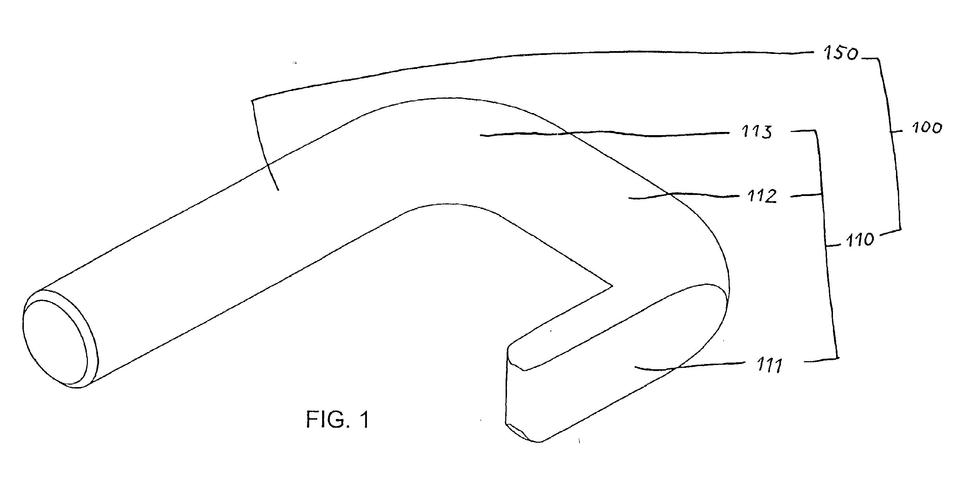

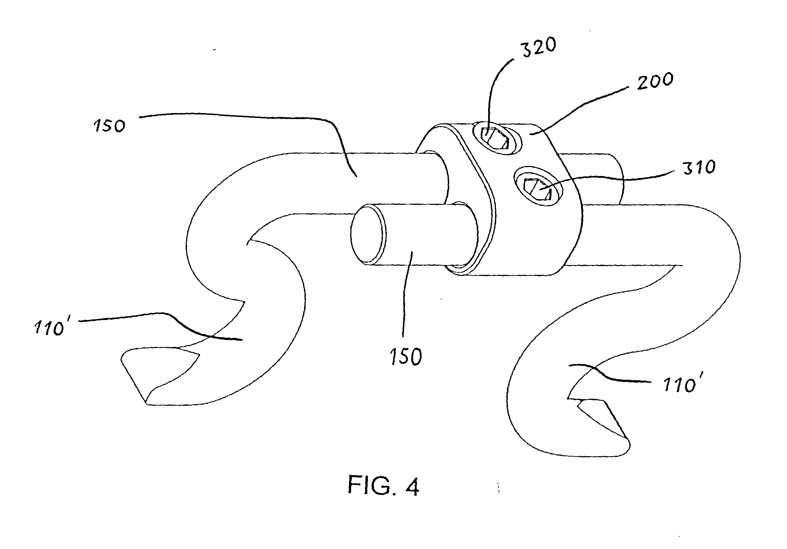

The spinal fixation hook of the present invention has a hooked portion which is provided with an extension rod for connecting with other element or member. The hooked portion is of any shape, such as U shape, as shown in FIG. 1, or S shape, as shown in FIG. 4.

The extension rod is preferably mad integrally with the hooked portion and is devoid of a fixation means. The extension rod is therefore passively fastened to an element or member having a fixation means.

The present invention also provides a spinal fixation retrieval device comprising:
a fixation seat having at least two through holes;
a rod-shaped object adapted to put through one of said at least two through holes of said fixation seat;
one or more said spinal fixation hooks of the present invention, wherein said extension rod is adapted to put through one of said at least two through holes of said fixation seat; and
one or more fixation means for fixing said rod-shaped object and said one or more spinal fixation hooks in said at least two through holes of said fixation seat.

Preferably, said one or more fixation means are a fixation screw.

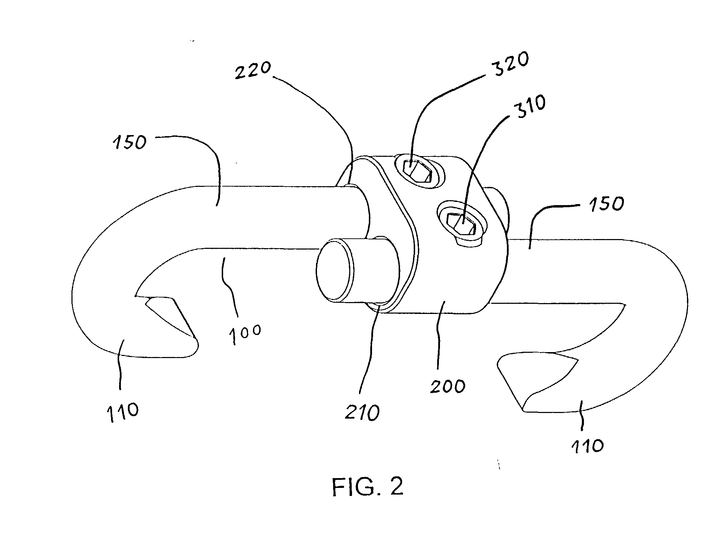

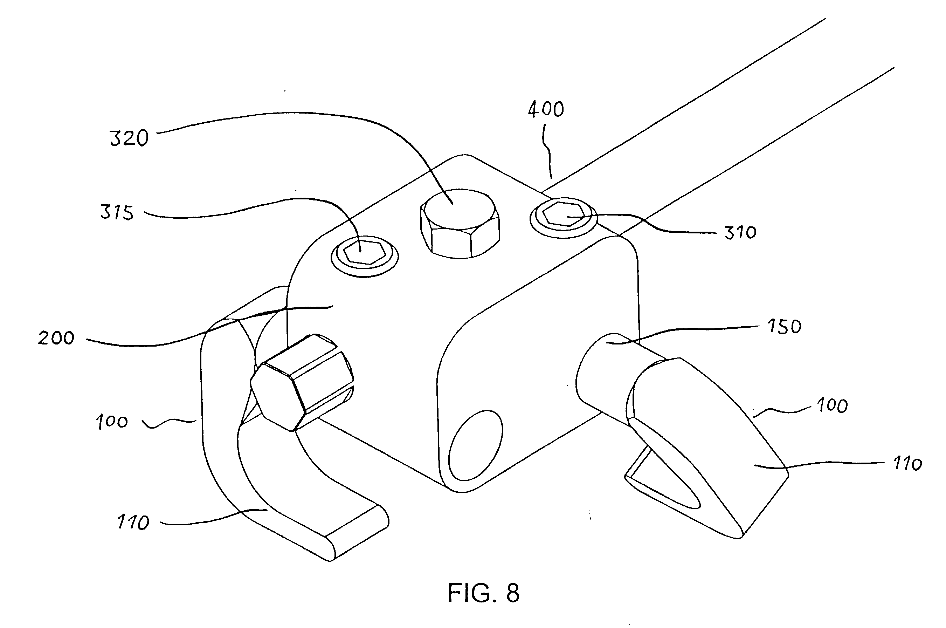

The fixation seat of the present invention may be an oval columnar construction. The through holes of the fixation seat of the present invention may be substantially parallel to one another, as shown in FIGS. 2-7. One of the through holes in which the rod-shaped object is received may be oriented in such a manner that it forms a specific angle to the other through holes, such as a right angle as shown in FIG. 8.

The rod-shaped object of the present invention may be a fixation rod of a fixation element or auxiliary fixation element, or the extension rod of the spinal fixation hook of the present invention shown in FIGS. 2-4.

The fixation means of the present invention are similar in construction to the conventional fixation means, such as the threaded fixation means or TRSH fixation system developed by Danek Group Inc., Medical Division, of the United States.

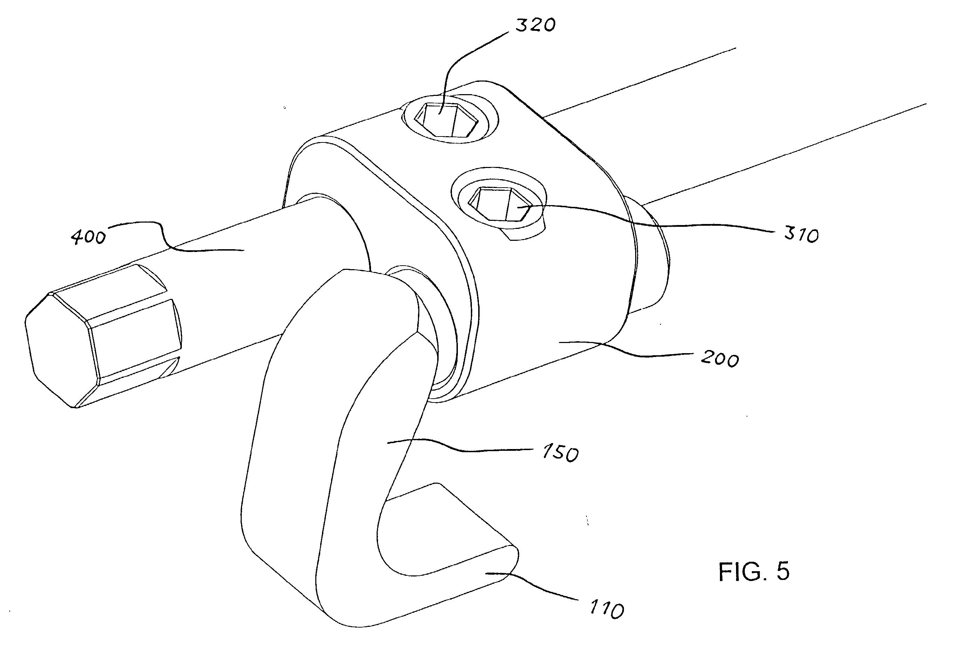

As shown in FIG. 1, a fixation hook 100 of the present invention has a hooked portion 110 and an extension rod 150. The hooked portion 110 has a hooked end 111, a curved portion 112, and a connection end 113. The hooked portion 110 is of a U-shaped construction shown in FIGS. 2-4, or an S-shaped construction shown in FIG. 5.

As shown in FIG. 2, a spinal fixation retrieval device according to a first embodiment of the present invention comprises a fixation seat 200, two fixation hooks 100 and two fixation screws 310 and 320 for use in fixing two fixation hook 100 with the fixation seat 200. The fixation seat 200 is provided with two through holes 210 and 220. In this embodiment, one of the two extension rods 150 of the fixation hook 100 is regarded as the rod-shaped object referred to above.

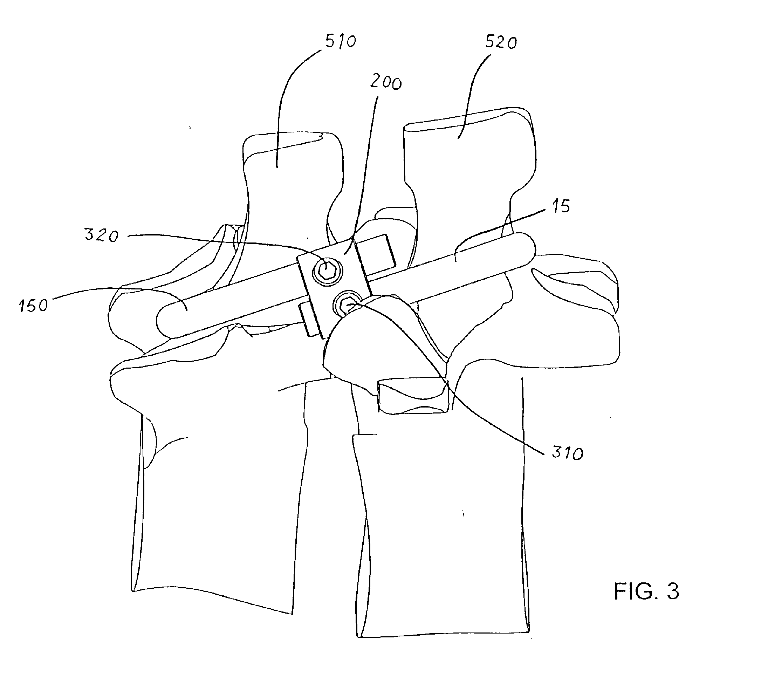

The spinal fixation retrieval device shown in FIG. 2 is used to fix two vertebrae 510 and 520, as shown in FIG. 3. A spinal fixation retrieval device according to a second embodiment of the present invention similar to the first embodiment in FIG. 2 is shown in FIG. 4, wherein like elements and parts are represented by like numerals. In this second embodiment, the fixation hooks have an S-shaped hooked portions 110'. In a third embodiment of the present invention, one of the two spinal fixation hooks 100 in FIG. 2 is replaced by a fixation rod 400 as shown in FIG. 5, where like elements and parts are represented by like numerals.

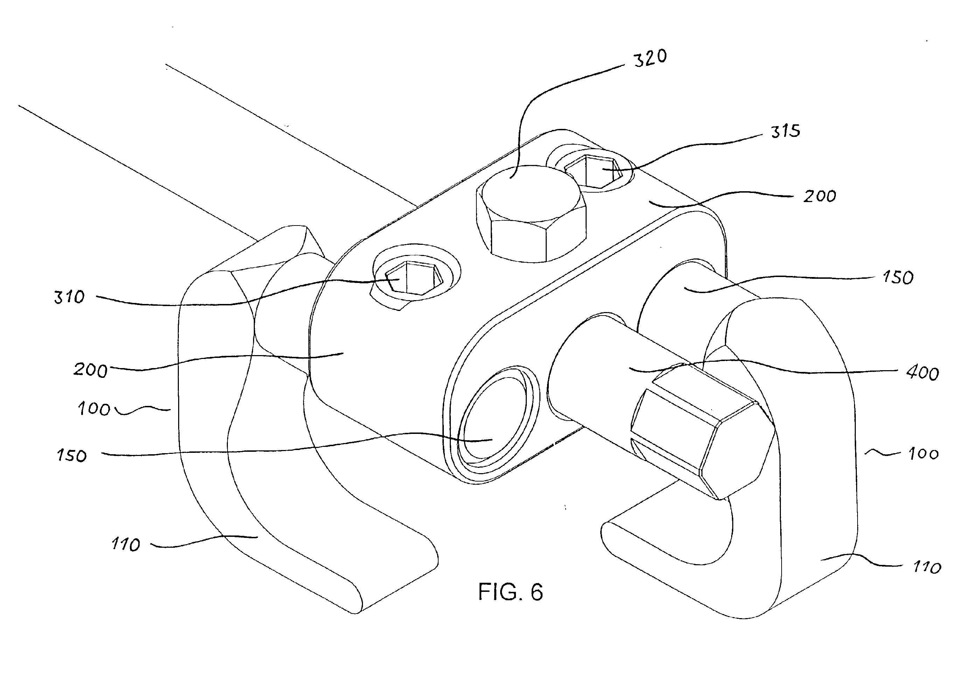

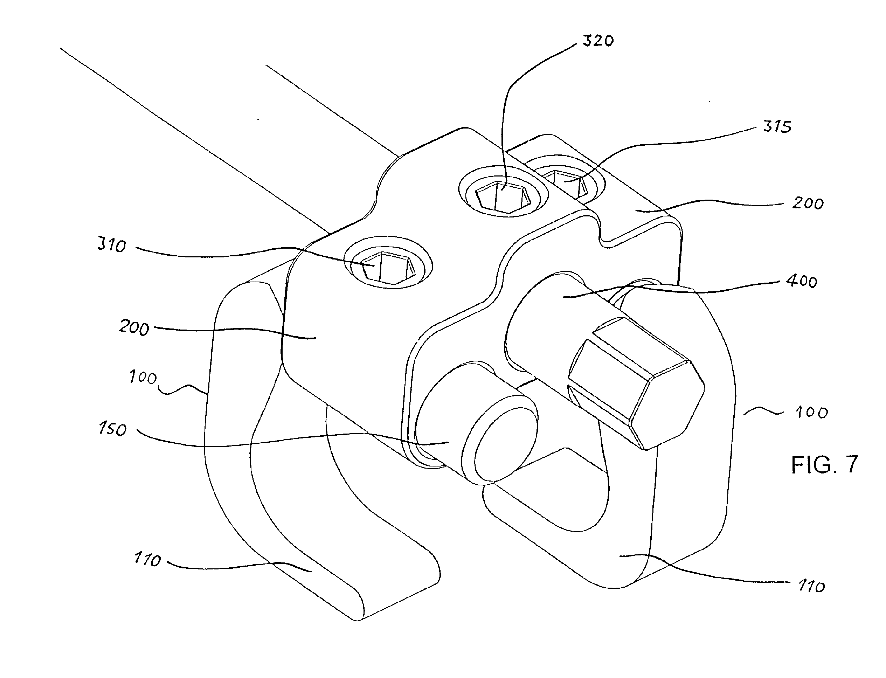

A fourth and fifth embodiments similar to the third embodiment shown in FIG. 5 are shown in FIGS. 6 and 7, respectively, wherein like elements and parts are represented by like numerals. In the fourth embodiment, an additional fixation hook 100 is fixed in an additional through hole of the fixation seat 200 by using an additional fixation screw 315. One significant difference between the fourth and fifth embodiments is the fixation rod 400 and two extension rods 150 of the two fixation hook 100 are substantially coplanar in the former, and they are not coplanar in the later, as shown in FIGS. 6 and 7, respectively. The two fixation hooks 100 are used to fix one vertebra.

A sixth embodiment similar to the fourth embodiment shown in FIG. 6 is shown in FIG. 8, wherein like elements and parts are represented by like numerals. As shown in FIG. 8, the extension rods 150 of the two fixation hooks 100 are substantially parallel to one another. The two extension rods 150 and the fixation rod 400 are not coplanar, with the fixation rod 400 being parallel to an imaginary plane on which the extension rods 150 are located. In addition, the fixation rod 400 is substantially perpendicular to the two extension rods 150.

## Claims

1. A spinal fixation retrieval device comprising:
a fixation seat having at least two through holes;
a rod-shaped object adapted to put through one of said at least two through holes of said fixation seat;
one or more fixation hooks, with each having an extension rod and a hooked portion, wherein said extension rod is adapted to put through one of said at least two through holes of said fixation seat; and
one or more fixation means for fixing said rod-shaped object and said one or more fixation hooks in said at least two through holes of said fixation seat.

2. The device as defined in claim 1, wherein said rod-shaped object is a fixation rod or an extension rod of a fixation hook.

3. The device as defined in claim 1, wherein said one or more fixation means are a fixation screw.

4. A spinal fixation hook comprising an extension rod and a hooked portion at one end of said extension rod.

5. The hook as defined in claim 4, wherein said extension rod is a straight rod.

6. The hook as defined in claim 4, wherein said hooked portion has a U shape, an S shape, a shape like the U shape, or a shape like the S shape.

7. The hook as defined in claim 5, wherein said hooked portion has a U shape, an S shape, a shape like the U shape, or a shape like the S shape.

## Amended claims

### Amended claims in accordance with Rule 86(2) EPC.

**1.** A spinal fixation retrieval device comprising:
a fixation seat (200) having at least two through holes (210, 220);
a rod-shaped object (150) adapted to put through one of said at least two through holes of said fixation seat;
one or more fixation hooks (100, 400), with each having an extension rod (150) and a hooked portion (110, 110'), wherein said extension rod is adapted to put through one of said at least two through holes of said fixation seat; and
one or more fixation means (310, 320, 315) for fixing said rod-shaped object and said one or more fixation hooks in said at least two through holes of said fixation seat.

**2.** The device as defined in claim 1, wherein said rod-shaped object (150) is a fixation rod or an extension rod of a fixation hook (100, 400).

**3.** The device as defined in claim 1, wherein said one or more fixation means (310, 320, 315) are a fixation screw.

**4.** The device as defined in any one of claims 1-3, wherein said hooked portion (100, 100') has a U shape, an S shape, a shape like the U shape, or a shape like the S shape.
